# EUROPÄISCHE PATENTANMELDUNG

(11) **EP 0 821 235 A2**
(43) Veröffentlichungstag der Anmeldung: **28.01.1998**
(21) Anmeldenummer: 97112362.5
(22) Anmeldetag: 18.07.1997
(51) Int. Cl.: G01N 33/52

(54) **Diagnostischer Testträger mit Kapillarspalt**

(30) Priorität: 23.07.1996 DE 19629654
(71) Anmelder: BOEHRINGER MANNHEIM GMBH, 68298 Mannheim (DE)
(72) Erfinder: Vetter, Peter, 67105 Schifferstadt (DE); Macho, Heinz, 64658 Fürth (DE); Vogel, Peter, Dr., 69502 Hemsbach (DE); Fritz, Michael, 68647 Biblis (DE)

(57) **Zusammenfassung**

Gegenstand der Erfindung ist ein diagnostischer Testträger (1) enthaltend eine Tragschicht (2) mit einer darauf angeordneten zur Bestimmung eines Analyten in flüssiger Probe erforderliche Reagenzien enthaltenden Nachweisschicht (3) und eine die Nachweisschicht (3) überdeckende inerte Schicht (4), wobei die inerte Schicht (4) in einem solchen Abstand (5) von der Nachweisschicht angeordnet ist, daß zwischen Nachweis- und inerter Schicht (3,4) ein kapillaraktiver Flüssigkeitstransport möglich ist,
dadurch gekennzeichnet, daß
die inerte Schicht (4) über die Nachweisschicht hinausreicht und in dem die Nachweisschicht überragenden Bereich (10) so auf der Tragschicht (2) befestigt ist, daß ein durchgehender kapillaraktiver Flüssigkeitstransport zwischen inerter und Nachweisschicht (4,3) und zwischen inerter und Tragschicht (4,2) möglich ist und außerdem die inerte Schicht (4) im Bereich der Nachweisschicht (3) mehrere Löcher (7) enthält, durch die die zu untersuchende Probe und darin enthaltene Inhaltsstoffe auf die Nachweisschicht (3) aufgegeben werden kann
sowie dessen Verwendung zur Bestimmung eines Analyts in einer Flüssigkeit.

## Beschreibung

Die Erfindung betrifft einen diagnostischen Testträger enthaltend eine Tragschicht mit einer darauf angeordneten, zur Bestimmung eines Analyts in flüssiger Probe erforderliche Reagenzien enthaltenden Nachweisschicht und eine die Nachweisschicht überdeckende inerte Schicht, wobei die inerte Schicht in einem solchen Abstand von der Nachweisschicht angeordnet ist, daß zwischen Nachweis- und inerter Schicht ein Spalt vorhanden ist, der einen kapillaraktiven Flüssigkeitstransport ermöglicht. Außerdem richtet sich die Erfindung auf die Verwendung eines solchen diagnostischen Testträgers zur Bestimmung eines Analyts in einer Flüssigkeit.

Zur qualitativen oder quantitativen analytischen Bestimmung von Bestandteilen von Körperflüssigkeiten, insbesondere von Blut, werden oft sogenannte tragergebundene Tests verwendet. Bei diesen sind Reagenzien in entsprechenden Schichten eines festen Testträgers eingebettet, der mit der Probe in Kontakt gebracht wird. Die Reaktion von flüssiger Probe und Reagenzien führt zu einem nachweisbaren Signal, insbesondere einem Farbumschlag, welcher visuell oder mit Hilfe eines Geräts, meistens reflektionsphotometrisch, ausgewertet werden kann.

Testträger sind häufig als Teststreifen ausgebildet, die im wesentlichen aus einer länglichen Tragschicht aus Kunststoffmaterial und daraufangebrachten Nachweisschichten als Testfeldern bestehen. Es sind jedoch auch Testträger bekannt, die als quadratische oder rechteckige Plättchen gestaltet sind.

Testträger der eingangs bezeichneten Art sind beispielsweise aus EP-A-0 348 006 bekannt. Dort soll vor allem eine gleichmäßige Verteilung der Probenflüssigkeit über den Testbezirk erreicht werden, der einen Teil der die zur Bestimmung des Analyten erforderlichen Reagenzien enthaltenden Nachweisschicht darstellt. Die gestellte Aufgabe wird nach den in der europäischen Patentanmeldung gemachten Angaben dadurch gelöst, daß sich über dem Testbezirk ein kapillarer Spalt befindet. Dieser ist nach Probenaufgabe und bei der Vermessung des Tests mit Flüssigkeit gefüllt (siehe beispielsweise Seite 6, Zeilen 3 bis 6 oder Fig. 5). Der Kapillarspalt und das Absorptionsvolumen des Testbezirks definieren somit das Probenvolumer, das von dem Testträger aufgenommen und dort untersucht wird. Ein wesentlicher Nachteil dieses Testträgers des Standes der Technik besteht darin, daß während der Zeit, die zur Durchführung der Bestimmungsreaktion erforderlich ist und bei der Analyt verbraucht wird, ständig Analyt aus der umgebenden Flüssigkeit nachdifundiert und so das Ergebnis verfälscht.

Ebenso ist aus EP-A-0 297 389 ein diagnostischer Testträger bekannt, bei dem nebeneinander eine Flüssigkeitstransportzone und eine Nachweiszone auf einer Tragschicht angeordnet sind, welche durch einen Kapillarspalt miteinander verbunden sind. Die Konstruktion des Testträgers führt dazu, daß bei undosierter Probenaufgabe und der damit erforderlichen überschüssigen Probenflüssigkeit der gesamte Kapillarspalt mit Flüssigkeit gefüllt ist. Auch hier wird dann Analyt, der in der Nachweiszone durch die Nachweisreaktion verbraucht wird, durch nachdiffundierenden Analyt aus umgebender Flüssigkeit ersetzt, wobei das Resultat positiv verfälscht werden kann.

Aufgabe der vorliegenden Erfindung ist es deshalb, einen diagnostischen Testträger zur Bestimmung eines Analyts in einer Flüssigkeit zur Verfügung zu stellen, auf den eine undosierte Probenflüssigkeitsmenge aufgegeben werden kann und bei dem ein Probenflüssigkeitsüber schuß nicht zu falsch-positiven Resultaten durch Nachdiffusion von Analyt aus umgebender Flüssigkeit führt.

Diese Aufgabe wird durch die in den Patentansprüchen naher charakterisierte Erfindung gelöst.

Gegenstand der Erfindung ist nämlich ein diagnostischer Testträger, der auf einer Tragschicht eine Nachweisschicht zur Bestimmung eines Analyts in flüssiger Probe enthält. Die Nachweisschicht enthält erforderliche Reagenzien für die Detektion des Analyts. Die Nachweisschicht ist von einer inerten Schicht überdeckt, wobei zwischen der inerten Schicht und der Nachweisschicht ein Spalt ausgebildet ist, der so groß ist, daß zwischen Nachweis- und inerter Schicht in kapillaräktiver Flüssigkeitstransport möglich ist. Die inerte Schicht ist größer als die Nachweisschicht und reicht deshalb über letztere hinaus. In dem die Nachweisschicht überragenden Bereich ist die inerte Schicht so aufder Tragschicht befestigt, daß sich auch hier ein Spalt zwischen Tragschicht und inerter Schicht befindet, der einen kapillaraktiven Flüssigkeitstransport zwischen diesen Schichten ermöglicht. Da erfindungsgemäß im gesamten diagnostischen Testträger die inerte Schicht in einem kleinen Abstand von der darunter befindlichen Schicht angeordnet ist, ist ein durchgehender kapillaraktiver Flüssigkeitstransport zwischen inerter und Nachweisschicht und zwischen inerter und Tragschicht möglich. Im Bereich der Nachweisschicht weist die inerte Schicht mehrere Löcher auf, durch die die zu untersuchende Probe und darin enthaltene Inhaltsstoffe auf die Nachweisschicht aufgegeben werden können.

Für die Tragschicht des erfindungsgemäßen diagnostischen Testträgers kommen insbesondere solche Materialien in Frage, die die zu untersuchenden Flüssigkeiten nicht aufnehmen. Dies sind sogenannte nicht-saugfähige Materialien, wobei Kunststoffolien, wie zum Beispiel Polyesterfolien, besonders bevorzugt sind.

Für die Nachweisschicht ist es im Gegensatz hierzu erforderlich, solche Materialien einzusetzen, die in der Lage sind, die zu untersuchende Flüssigkeit mit darin enthaltenen Inhaltsstoffen aufzunehmen. Dies sind sogenannte saugfähige Materialien, wie beispielsweise Papiere, Vliese, Gewebe, Gewirke oder poröse Kunststoffmaterialien, die als Schichtmaterialien verwendet werden können. Die für die Nachweisschicht in Frage kommenden Materialien müssen natürlich auch Reagenzien tragen können, die für den Nachweis des zu bestimmenden Analyts erforderlich sind. Im einfachsten Fall befinden sich alle für den Nachweis des Analyts erforderliche Reagenzien auf einer Schicht. Es sind jedoch auch Fälle vorstellbar, für die es vorteilhafter ist, die Reagenzien aufmehrere saugfähige Materialschichten zu verteilen, die dann übereinander sich vollflächig berührend angeordnet sind. Der im folgenden verwendete Begriff "Nachweisschicht" soll sowohl solche Falle umfassen, bei denen sich die Reagenzien entweder nur in oder auf einer Schicht oder in zwei oder noch mehr, wie vorstehend beschrieben, angeordneten Schichten befinden. Bevorzugte Materialien für die Nachweisschicht sind Papiere oder poröse Kunststoffmaterialien wie Membranen. Besonders bevorzugt sind asymmetrisch poröse Membranen, die so angeordnet sind, daß die zu untersuchende Probenflüssigkeit auf die großporige Seite der Membran aufgegeben wird und die Bestimmung des Analyts von der feinporigen Seite der Membran aus erfolgt. Als poröse Membranmaterialien sind Polyamid-, Polyvinylidendifluorid-, Polyethersulfon- oder Polysulfonmembranen ganz besonders bevorzugt. Für den erfindungsgemäßen Testträger sind insbesondere Polyamid 66-Membranen hervorragend geeignet. Die Reagenzien zur Bestimmung des nachzuweisenden Analyts sind in der Regel durch Imprägnierung in die vorstehend genannten Materialien eingebracht worden. Für die Nachweisschicht kommen jedoch auch sogenannte offene Filme in Frage, wie sie beispielsweise in EP-B-0 016 387 beschrieben sind. Hierfür werden einer wäßrigen Dispersion von filmbildenden organischen Kunststoffen Feststoffe als feine, unlösliche, organische oder anorganische Partikel zugegeben und die für die Nachweisreaktion erforderlichen Reagenzien zusätzlich hinzugefügt. Geeignete Filmbildner sind bevorzugt organische Kunststoffe, wie Polyvinylester, Polyvinylacetate, Polyacrylester, Polymethacrylsäure, Polyacrylamide, Polyamide, Polystyrol, Mischpolymerisate, zum Beispiel von Butadien und Styrol oder von Maleinsäureester und Vinylacetat oder andere filmbildende, natürliche und synthetische organische Polymere sowie Mischungen derselben in Form von wäßrigen Dispersionen. Die Dispersionen lassen sich auf einer Unterlage zu einer gleichmäßigen Schicht verstreichen, die nach dem Trocknen einen wasserfesten Film ergibt. Die trockenen Filme haben eine Dicke von 10 µm bis 500 µm, vorzugsweise von 30 bis 200 µm. Der Film kann mit der Unterlage als Träger zusammen verwendet werden oder für die Nachweisreaktion auf einen anderen Träger aufgebracht werden. Obwohl die für die Nachweisreaktion erforderlichen Reagenzien normalerweise in die zur Herstellung der offenen Filme verwendete Dispersion gegeben werden, kann es auch vorteilhaft sein, wenn der gebildete Film nach seiner Herstellung mit den Reagenzien imprägniert wird. Auch eine Vorimprägnierung der Füllstoffe mit den Reagenzien ist möglich. Welche Reagenzien zur Bestimmung eines bestimmten Analyts eingesetzt werden können, sind dem Fachmann bekannt. Dies muß hier nicht näher ausgeführt werden.

Außerdem kann die Nachweisschicht auch eine Schicht enthalten, die Plasma oder Serum aus Vollblut abzutrennen in der Lage ist, wie beispielsweise ein Glasfaservlies, wie es aus EP-B-0 045 476 bekannt ist. Eine oder mehrere solcher Abtrennschichten kann über einer oder mehreren Schichten, die Nachweisreagenzien tragen, liegen. Auch ein solcher Aufbau soll von dem Begriff "Nachweisschicht" umfaßt sein.

Für die inerte Schicht des erfindungsgemäßen diagnostischen Testträgers kommen dünne, im wesentlichen wasserundurchlässige Materialien in Frage. Kunststoffolien haben sich hierfür als besonders gut geeignet erwiesen. Damit die zu untersuchende Probenflüssigkeit durch die inerte Schicht auf die Nachweisschicht gelangen kann, enthält die inerte Schicht im Bereich der Nachweisschicht Löcher. Diese Löcher können alle möglichen Formen haben. Sie können beispielsweise rechteckig, quadratisch, vieleckig oder rund sein. Runde Löcher haben sich aus herstellungstechnischen Gründen als bevorzugt erwiesen. Die Löcher können gleichmäßig angeordnet sein, sie können aber auch rein statistisch verteilt sein. Die Löcher können gleich groß oder unterschiedlich groß sein. Aus herstellungstechnischen Gründen hat es sich als bevorzugt erwiesen, daß die Löcher gleich groß sind und in einem regelmäßigen Muster angeordnet sind. Ganz besonders bevorzugt sind die Löcher in gleichmäßig voneinander angeordneten parallelen Reihen in die inerte Folie gebracht. Im Bereich der Nachweisschicht weist die inerte Schicht ein Raster von Löchern auf, wobei die Lochfläche 30 bis 80, vorzugsweise 40 bis 70% der Nachweisschichtfläche ausmacht. In einer ganz besonders bevorzugten Ausführungsform entspricht der kleinste Abstand der Löcher dem Lochdurchmesser. Die Lochgröße, das heißt, der größte Lochdurchmesser beträgt etwa 0,5 bis 2 mm, vorzugsweise 0,6 bis 1,5 mm.

Der für den erfindungsgemäßen diagnostischen Testträger charakteristische kapillare Flüssigkeitstransport zwischen inerter- und Nachweisschicht sowie zwischen inerter- und Tragschicht wird dadurch erreicht, daß die jeweiligen Schichten in einem geringen Abstand übereinander angeordnet sind. Vorzugsweise ist dieser Abstand zwischen inerter- und Tragschicht geringer als der Abstand zwischen inerter- und Nachweisschicht. Die Größenordnung des Abstandes beträgt etwa zwischen 0,02 und 1,2 mm, vorzugsweise 0,4 - 1 mm. Zur Wahrung eines bestimmten Abstandes zwischen den jeweiligen Schichten über die gesamte Breite und Länge des in Frage kommenden Bereiches des erfindungsgemäßen diagnostischen Testträgers hat es sich als vorteilhaft erwiesen, daß Abstandshalter zwischen den Schichten angeordnet sind. Ganz besonders bevorzugt sind dafür diskrete Schmelzkleberbereiche geeignet, wie sie beispielsweise aus der europäischen Patentanmeldung 0 297 389 bekannt sind. Als Schmelzkleber können handelsübliche Produkte, beispielsweise auf Basis von Ethylen-Vinylacetat-Copolymeren, Polyestern oder Polyamiden zum Einsatz kommen. Die als Abstandshalter dienenden Schmelzkleberbereiche können sehr verschiedene Formen haben. Sie müssen jedenfalls so gestaltet sein, daß ausreichend große Zwischenräume bleiben, so daß ein Flüssigkeitstransport zwischen ihnen hindurch möglich ist. Bevorzugt bedecken sie zwischen 10 und 70%, besonders bevorzugt zwischen 15 und 50% der gesamten Fläche zwischen den jeweiligen, auf Abstand zu haltenden Schichten. Als sehr vorteilhaft hat es sich erwiesen, wenn der Schmelzkleber in Form von Partikeln, also beispielsweise in Form von runden, quadratischen oder vieleckigen Punkten als Abstandshalter dient. In einer bevorzugten Ausführungsform des diagnostischen Testträgers sind die Schmelzkleberbereiche auf der inerten Schicht befestigt. Sie liegen auf der Nachweisschicht nur auf und sind dort nicht auch befestigt. Außerhalb der Nachweisschicht jedoch sind die Schmelzkleberbereiche sowohl an der inerten Schicht als auch an der Tragschicht befestigt. Um einen möglichst gleichmäßigen Spaltabstand zwischen den beiden durch die Schmelzkleberbereiche verbundenen Schichten sicherzustellen, sind die Schmelzkleberbereiche bevorzugt gleich groß und gleichmäßig über die gesamte Verbindungsfläche verteilt. Bevorzugt haben die Schmelzkleberpartikel eine durchschnittliche Grundfläche zwischen 0,03 und 5,0 mm². Besonders bevorzugt beträgt die Obergrenze 0,1 mm².

Zur Bestimmung des in der Proberflüssigkeit nachzuweisenden Analyten ist in dem erfindungsgemäßen diagnostischen Testträger die Nachweisschicht durch die Tragschicht sichtbar. Dies kann dadurch erreicht werden, daß die Tragschicht transparent ist. Es ist aber auch möglich, daß die Tragschicht ein Loch aufweist, das von der Nachweisschicht überdeckt ist. Durch das Loch ist dann die Nachweisschicht sichtbar. In einer bevorzugten Ausführungsform des erfindungsgemäßen diagnostischen Testträgers befindet sich in der Tragschicht unterhalb der Nachweisschicht ein Loch, durch das die Nachweisschicht beobachtbar ist. Das Loch besitzt einen etwas kleineren Durchmesser als die kleinste Längenausdehnung der Nachweisschicht, so daß die Nachweisschicht außerhalb des Loches aufder Tragschicht aufliegt und dort befestigt sein kann. Beispielsweise hat es sich als vorteilhaft erwiesen, daß die Nachweisschicht mittels einer dünnen Schmelzkleberschicht auf der Tragschicht befestigt ist.

Die inerte Folie wird vor dem Aufbringen auf die Nachweis- und Tragschicht mit Schmelzkleberbereichen versehen. Beispielsweise kann der Schmelzkleber durch Siebdruck aufgebracht werden. Andere Methoden sind dem Fachmann beispielsweise aus der europäischen Patentanmeldung 0 297 389 bekannt. So wird dort ein Verfahren beschrieben, bei dem Schmelzkleber mittels einer Breitschlitzdüse durch die Löcher eines Siebes hindurchgedrückt wird, wobei die Form der dabei erzeugten Schmelzkleberpartikel durch die Form der Löcher des Siebes bestimmt ist. Da der Schmelzkleber beim Aufbringen auf die inerte Folie noch so warm ist, daß er in geschmolzenen Zustand die inerte Schicht berührt, haftet er dort beim Erkalten fest an.

Nach dem Aufbringen der Schmelzkleberbereiche können Löcher in die inerte Schicht gemacht werden, die im späteren erfindungsgemäßen diagnostischen Testträger im Bereich der Nachweisschicht angeordnet sind. Die so vorbereitete inerte Schicht wird auf die Tragschicht und die darauf befindliche Nachweisschicht aufgelegt. Die außerhalb der Nachweisschicht befindlichen Schmelzkleberbereiche werden anschließend erneut so angewärmt, daß die Bereiche zumindest oberflächlich zum Schmelzen kommen. Durch Druck der inerten Schicht auf die Tragschicht außerhalb des Bereiches der Nachweisschicht wird die inerte Schicht auf der Tragschicht befestigt. Der Druck darf hierbei nicht so groß sein, daß kein Abstand mehr zwischen inerter Schicht und Tragschicht existiert. In diesem Fall wäre kein kapillaraktiver Flüssigkeitstransport mehr möglich.

Eine bevorzugte Ausführungsform des erfindungsgemäßen diagnostischen Testträgers und diesen Testträger betreffende Details sind in Figuren 1-5 dargestellt.

Fig. 1 stellt einen Querschnitt durch einen bevorzugten erfindungsgemäßen diagnostischen Testträger dar.

Fig.2 zeigt die Aufsicht auf den in Fig. 1 im Querschnitt dargestellten erfindungsgemäßen diagnostischen Testträger.

Fig.3 zeigt den vergrößerten Ausschnitt des in Fig. 1 zwischen A und B dargestellten Querschnitts.

Fig.4 stellt das Detail des in Fig.3 zwischen C und D dargestellten Querschnitts dar.

Fig.5 zeigt eine Aufsicht aufeinen Teil einer inerten Schicht mit Abstandshaltern, wie sie im erfindungsgemäßen diagnostischen Testträger der Tragschicht zugewandt sind.

Die in den Figuren verwendeten Bezugszeichen bedeuten:
- 1: diagnostischer Testträger
- 2: Tragschicht
- 3: Nachweisschicht
- 4: inerte Schicht
- 5: Abstand zwischen Nachweis- und inerter Schicht
- 6: Abstand zwischen Trag- und inerter Schicht
- 7: Löcher in der inerten Schicht im Bereich der Nachweisschicht
- 8.: Abstandshalter
- 9.: Zwischenraum zwischen Abstandshaltern
- 10.: über die Nachweisschicht hinausragender Bereich der inerten Schicht
- 11.: Loch unter Nachweisschicht in der Tragschicht
- 12.: Richtungspfeile auf inerter Schicht
- 13.: Positionierloch

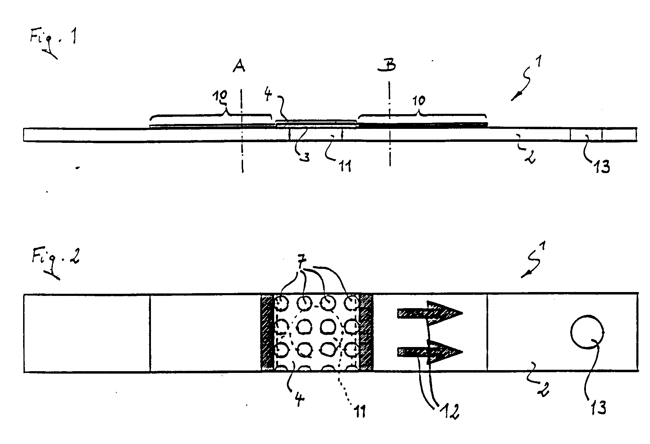

Der in Fig. 1 dargestellte Querschnitt durch einen erfindungsgemäßen diagnostischen Testträger (1) zeigt eine Tragschicht (2) mit der Nachweisschicht (3) und der die Nachweisschicht (3) überdeckenden inerten Schicht (4), die im über die Nachweisschicht (3) hinausragenden Bereich (10) auf der Tragschicht (2) befestigt ist. Unter der Nachweisschicht (3) befindet sich ein Loch (11) in der Tragschicht (2) durch das die Nachweisschicht (3) sichtbar ist. Das Positionierloch (13) dient dazu, den Teststreifen im Falle einer apparativen, beispielsweise einer reflektionsphotometrischen Vermessung an einer genau vorbestimmten Stelle des Apparates festzuhalten. Dies kann dadurch geschehen, daß beispielsweise ein Stift in das Positionierloch (13) hineinragt und so den Teststreifen (1) an einer bestimmten Stelle festhält.

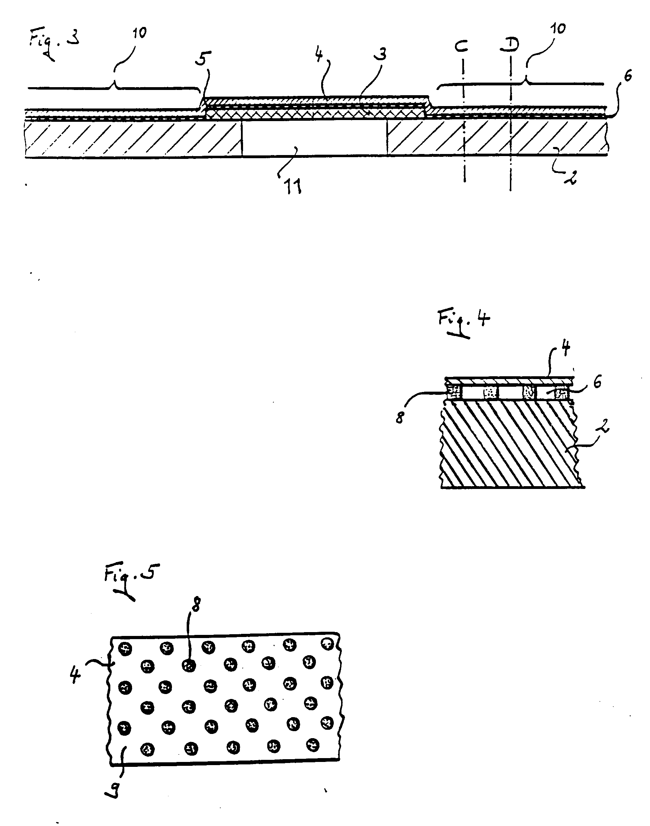

In der in Fig. 2 gezeigten Aufsicht aufden erfindungsgemäßen diagnostischen Testträger (1) erkennt man die Löcher (7), die sich im Bereich der Nachweisschicht (3) in der inerten Schicht (4) befinden. Das Loch (11) in der Tragschicht (2) ist in dieser Figur gestrichelt dargestellt. Die Richtungspfeile (12) auf der inerten Schicht (4) außerhalb des Bereiches der Nachweisschicht (3) zeigen dem Benutzer im Falle der Verwendung eines Gerätes zur apparativen Bestimmung des nachzuweisenden Analyts in welcher Richtung der Teststreifen in das Gerät eingeführt werden soll. Zur Positionierung im Gerät hilft dann, wie bereits vorstehend erläutert, das Positionierloch (13) am Ende des Testträgers (1).

Aus dem in Fig. 3 gezeigten, vergrößerten Ausschnitt des in Fig. 1 zwischen A und B dargestellten Querschnitts ist der Abstand (5) zwischen inerter Schicht (4) und Nachweisschicht (3) einerseits und der Abstand (6) zwischen inerter Schicht (4) und Tragschicht (2) andererseits ersichtlich. Auch im Bereich der Kanten der Nachweisschicht (3) liegt die inerte Schicht (4) nicht so fest an, daß ein kapillaraktiver Flüssigkeitstransport aus dem Bereich der Nachweisschicht (4) in den über die Nachweisschicht (3) hinausragenden Bereich (10) der inerten Schicht (4) nicht möglich wäre.

Bei dem in Fig. 4 dargestellten Detail des in Fig. 3 zwischen C und D dargestellten Querschnitts sind die Abstandshalter (8) zwischen inerter Schicht (4) und Tragschicht (2) ersichtlich, die einen bestimmten Abstand (6) zwischen Tragschicht (2) und inerter Schicht (4) einstellen. Eine mögliche Anordnung der Abstandshalter (8) aufder inerten Schicht (4) ist in Fig. 5 dargestellt. Hier sind die Abstandshalter in parallelen diagonal zu der inerten Schicht (4) verlaufenden Reihen angeordnet. Natürlich sind auch andere Anordnungen möglich, so lange ein gleichmäßiger Abstand derjeweiligen Schichten gewährleistet werden kann und der Zwischenraum (9) zwischen den Abstandshaltern ausreichend groß ist, damit ein kapillaraktiver Flüssigkeitstransport zwischen den Schichten stattfinden kann.

Ein erfindungsgemäßer diagnostischer Testträger kann sehr vorteilhaft zur Bestimmung eines Analyts in einer Flüssigkeit eingesetzt werden. Hierzu wird die zu untersuchende Flüssigkeit, vorteilhafterweise insbesondere Körperflüssigkeit wie Blut, Plasma, Serum oder Urin auf die Löcher der inerten Schicht aufgegeben. Durch die Löcher gelangt Flüssigkeit auf die saugfähige Nachweisschicht. Außerdem gelangt Flüssigkeit durch die Löcher in den Zwischenraum zwischen inerter und Nachweisschicht und wird aufgrund der dort wirkenden Kapillarkräfte gleichmäßig verteilt. Insgesamt findet so ein sehr vorteilhafter Spreiteffekt statt, so daß die Nachweisschicht insgesamt gleichmäßig und einheitlich Flüssigkeit aufnehmen kann. Überschüssige Flüssigkeit, die nicht mehr von der Nachweisschicht aufgenommen werden kann, wird durch den Zwischenraum zwischen inerter und Tragschicht von der Nachweisschicht weggesaugt. Hierdurch wird gewährleistet, daß das Saugvolumen der Nachweisschicht das zur Untersuchung gelangende Volumen der Probenflüssigkeit bestimmt. Dadurch, daß überschüssige Flüssigkeit von der Nachweisschicht wegtransportiert wird, wird verhindert, daß während der Nachweisreaktion und dem damit verbundenen Verbrauch von Analyt in der in der Nachweisschicht befindlichen Flüssigkeit weiterer Analyt in die Nachweisschicht gelangt und so das Ergebnis positiv verfälscht. Durch die Tragschicht hindurch kann die Nachweisschicht direkt beobachtet werden. Ein bei Anwesenheit des zu bestimmenden Analyts in der Probenflüssigkeit beispielsweise auftretender Farbwechsel oder eine Farbveränderung kann so visuell oder auch apparativ beobachtet werden. Dadurch, daß durch die Dimension und Materialbeschaffenheit der Nachweisschicht das Volumen der zu untersuchenden Probenflüssigkeit vorbestimmt werden kann und überschüssige Flüssigkeit nach Sättigung der Nachweisschicht abgesaugt wird und so störende Effekte vermieden werden, kam mit dem erfindungsgemäßen diagnostischen Testträger nicht nur eine qualitative, sondern auch eine quantitative Bestimmung von Analyten durchgeführt werden, ohne daß die zu untersuchende Probenflüssigkeit dosiert aufgegeben werden muß. Weil überschüssige Flüssigkeit von der Nachweisschicht weg in den Zwischenraum zwischen inerter und Tragschicht gesaugt wird, wird auch hygienischen Gesichtspunkten Rechnung getragen. Ein Herabtropfen von Flüssigkeit aus dem Testträger oder ein Kontakt von Flüssigkeit beispielsweise mit Teilen eines Gerätes, in das der Testträger zur apparativen Auswertung gelegt wird, wird zuverlässig vermieden. Dies ist insbesondere bei der Untersuchung von Blut oder von Blut abgeleiteten Proben wie Plasma oder Serum ein sehr wichtiger Aspekt.

Aufgrund des einfachen Aufbaus des erfindungsgemäßen Testträgers aus wenigen Materialien ist die Herstellung des Testträgers einfach und besonders kostengünstig.

### Beispiel 1

### Testträger zur Bestimmung von Hämoglobin in Blut

Ein Testträger gemäß Figur 1 wird hergestellt, indem eine asymmetrisch poröse Membran (BTS 65, Memtec America Corp., Timonium, Maryland, USA) mit einer Breite von 28 cm als Nachweisschicht (3) in einer Lösung von 1 Gew.-% Natriumdodeylsulfat, und 0,7 mmol/l Kaliumhexacyanoferrat (III) in 0,1 N Phosphatpuffer pH7 getränkt wird. Die flüssigkeitsbeladene Membran wird 30 Minuten bei 50° C getrocknet. Die trockene Membran wird in 6 mm breite Streifen geschnitten und auf eine 350 µm dicke, 48 mm breite Polyesterfolie Melinex 329 der Firma ICI) als Tragschicht (2) abgelegt. Diese Folie enthält 2 Reihen Löcher (11 und 13). In der ersten Reihe weisen die Löcher einen Durchmesser von 4 mm auf Sie sind 2,4 mm voneinander entfernt und der Mittelpunkt der Löcher ist jeweils 25 mm vom rechten Rand der Folie entfernt. In der zweiten Reihe von Löchern, die parallel zu der ersten Reihe angeordnet sind, besitzen die Löcher einen Durchmesser von 2,5 mm. Sie sind 3,5 mm voneinander entfernt und der Mittelpunkt der Löcher ist jeweils 4 mm vom rechten Folienrand entfernt. Die Membran wird so positioniert, daß sie mit ihrer feinporigen Seite über der ersten Reihe von Löchern (11) mit 4 mm Durchmesser zu liegen kommt.

Eine 50 µm dicke und 24 mm breite Hostaphan® FN50-Folie (Hoechst AG) als inerte Schicht (4), die im Rasterauftrag mit Schmelzkleberpunkten (Elvax 410 der Firma DuPont) (8) beschichtet ist, wird mit einer Rotationsstanze in der Mitte über eine Breite von 5,5 mm mit Löchern (7) eines Durchmessers von 1,5 mm versehen. Die Folie wird mit den Schmelzkleberpunkten so aufder Polyesterfolie positioniert, daß sich der gelochte Bereich über der Matrix befindet und die ungelochten Bereiche (10) links und rechts der Membran. Eine Heizwalze mit einer Spalteinstellung von 0,7 mm fixiert bei einer Temperatur zwischen 90 und 100° C die Hostaphan® -Folie auf der Polyesterfolie, so daß ein Kapillarspalt (9) zwischen den beiden Folien verbleibt. Anschließend wird das so erhaltene Band in 6 mm breite Testträger der Fig. 1 geschnitten.

Auf die Löcher (7) in der inerten Schicht (4) wird ca. 10 µm Vollblut aufgegeben. Der Teststreifen wird nach ca. 45 Sekunden durch das Loch (11) unter der Membran in der Tragschicht untersucht. Es wird eine gleichmäßige dunkle Färbung beobachtet, die mit dem Hämoglobingehalt des Blutes korreliert.

## Patentansprüche

1. Diagnostischer Testträger (1) enthaltend eine Tragschicht mit einer darauf angeordneten zur Bestimmung eines Analyten in flüssiger Probe erforderliche Reagenzien enthaltenden Nachweisschicht (3) und eine die Nachweisschicht (3) überdeckende inerte Schicht (4), wobei die inerte Schicht (4) in einem solchen Abstand (5) von der Nachweisschicht angeordnet ist, daß zwischen Nachweis- und inerter Schicht (3,4) ein kapillaraktiver Flüssigkeitstransport möglich ist,
dadurch gekennzeichnet, daß
die inerte Schicht (4) über die Nachweisschicht hinausreicht und in dem die Nachweisschicht überragenden Bereich (10) so auf der Tragschicht (2) befestigt ist, daß ein durchgehender kapillaraktiver Flüssigkeitstransport zwischen inerter und Nachweisschicht (4,3) und zwischen inerter und Tragschicht (4,2) möglich ist und außerdem die inerte Schicht (4) im Bereich der Nachweisschicht (3) mehrere Löcher (7) enthält, durch die die zu untersuchende Probe und darin enthaltene Inhaltsstoffe auf die Nachweisschicht (3) aufgegeben werden kann.

2. Diagnostischer Testträger (1) gemäß Patentanspruch 1, dadurch gekennzeichnet, daß die Nachweisschicht (3) aus flüssigkeitsaufnehmendem Material besteht.

3. Diagnostischer Testträger (1) gemäß Patentanspruch 1, dadurch gekennzeichnet, daß die inerte Schicht (4) aus Material besteht, das im wesentlichen keine Flüssigkeit aufnimmt.

4. Diagnostischer Testträger (1) gemäß Patentansprüchen 1-3, dadurch gekennzeichnet, daß die inerte Schicht (4) im Bereich der Nachweisschicht (3) ein Raster von Löchern (7) aufweist, wobei die Lochfläche 30-80, vorzugsweise 40-70% der Nachweisschichtfläche beträgt.

5. Diagnostischer Testträger gemäß Patentansprüchen 1-4, dadurch gekennzeichnet, daß der Abstand (6) zwischen inerter und Tragschicht (4, 2) geringer ist als der Abstand (5) zwischen inerter und Nachweisschicht (4,3).

6. Diagnostischer Testträger (1) gemäß einem der Patentansprüche 1-5, dadurch gekennzeichnet, daß zwischen Nachweis- und inerter Schicht (3,4) sowie zwischen Tragschicht (2) und inerter Schicht (4) Abstandshalter (8) einen solchen Abstand (5,6) der Schichten voneinander einstellen, daß dazwischen ein kapillaraktiver Flüssigkeitstransport möglich ist.

7. Diagnostischer Testträger (1) gemäß Patentanspruch 6, dadurch gekennzeichnet, daß eine Vielzahl diskreter Schmelzkleberbereiche als Abstandshalter (8) dienen.

8. Diagnostischer Testträger (1) gemäß Patentanspruch 7, dadurch gekennzeichnet, daß die Schmelzkleberbereiche auf der inerten Schicht (4) befestigt sind, auf der Nachweisschicht (3) aber nur lose aufliegen.

9. Diagnostischer Testträger (1) gemäß einem der Patentansprüche 1-8, dadurch gekennzeichnet, daß die Nachweisschicht (3) durch die Tragschicht (2) sichtbar ist.

10. Diagnostischer Testträger (1) gemäß Patentanspruch 9, dadurch gekennzeichnet, daß die Tragschicht (2) ein Loch (11) aufweist, das von der Nachweisschicht (3) überdeckt ist.

11. Verwendung eines diagnostischen Testträgers (1) gemäß einem der Patentansprüche 1-10 zur Bestimmung eines Analyts in einer Flüssigkeit.
